# EUROPEAN PATENT APPLICATION

(11) **EP 2 722 078 A1**
(43) Date of publication of application: **23.04.2014**
(21) Application number: 12189459.6
(22) Date of filing: 22.10.2012
(51) Int. Cl.: A63B 24/00, A61B 5/0488

(54) **Muscle activity traning facility for the upper body of a user**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A muscle activity training facility includes a monitor (12) coupled to a processor (11) and having a screen (15) which includes two areas (16, 17), and two figures (16) in one of the areas for indicating the standard operations of the muscles in front and rear portions of the user, and two further figures (20, 21) in the other area (17) for indicating the exercising of the muscles in the front and the rear portions of the user, a carrier (30) is attached to an upper body of the user, and a number of electrodes (40-45) are attached to the carrier (30) and coupled to the processor (11) for detecting and showing the activities of the muscles of the user in the other figures (20, 21) in the other area (17) of the screen (15).

## Description

The invention relates to a muscle activity training facility for sensing or detecting or training the muscle activities of the user.

Typical muscle activity training devices comprise one or more sensors or detectors or electrodes for detecting the muscle activities of the muscles.

However, the sensors or detectors or electrodes are attached to the hairy portions of the user with tapes or adhesive materials and may have a good chance to be disengaged from the user after use.

The invention is to provide a muscle activity training facility for detecting or training the muscle activities of the user.
Fig. 1 is a plan view of a muscle activity training facility;
Fig. 2 is a plan view of a display screen of the facility;
Fig. 3 is a lower perspective view of a carrier;
Figs. 4, 5 are partial front views of a front portion and a rear portion of a user;
Figs. 6, 7, 8, 9 are plan views illustrating the display screen of the muscle activity training facility.

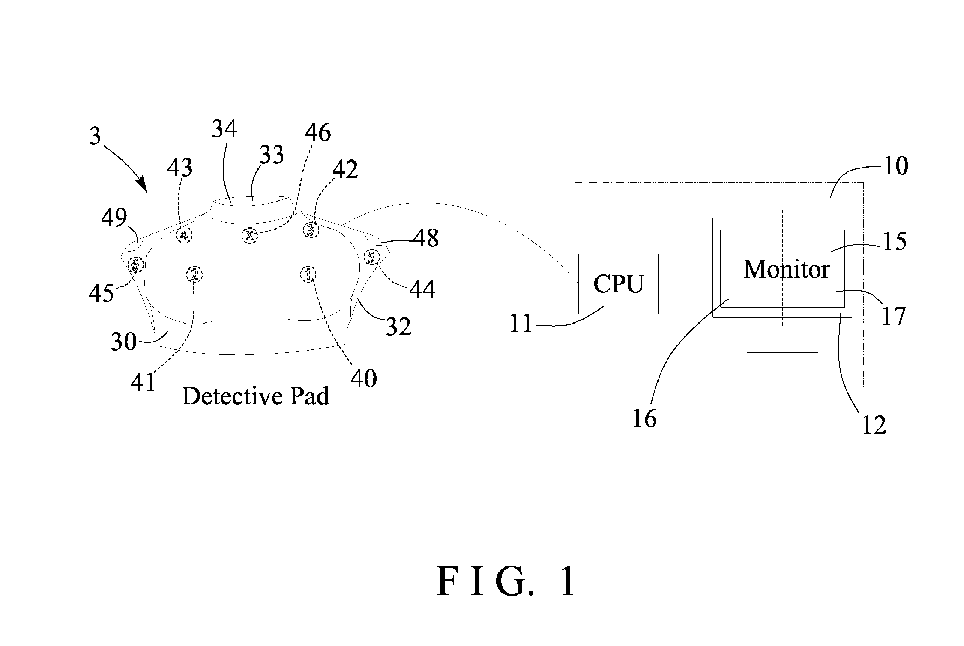

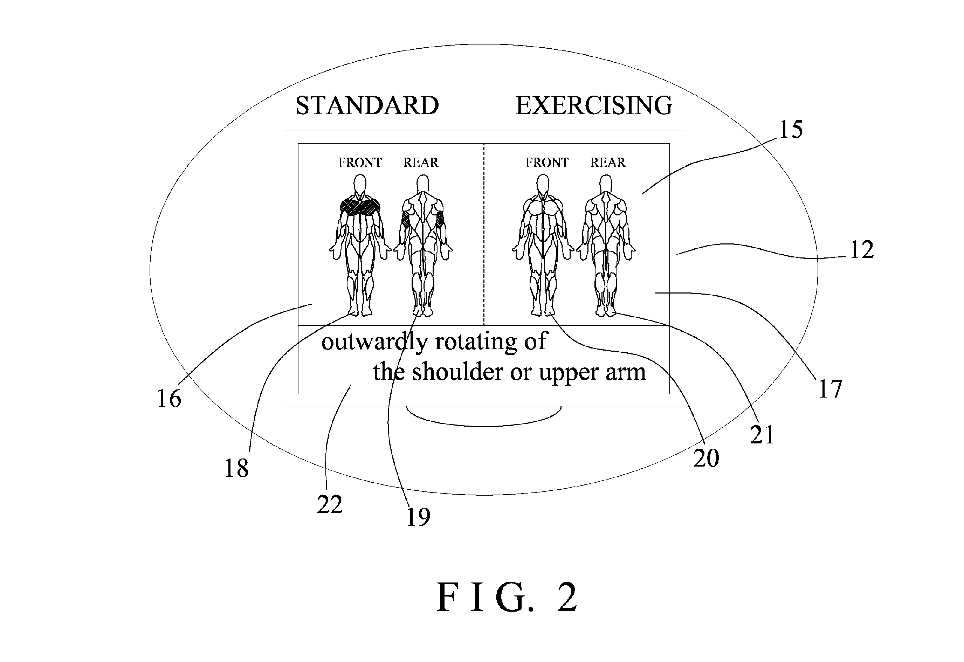

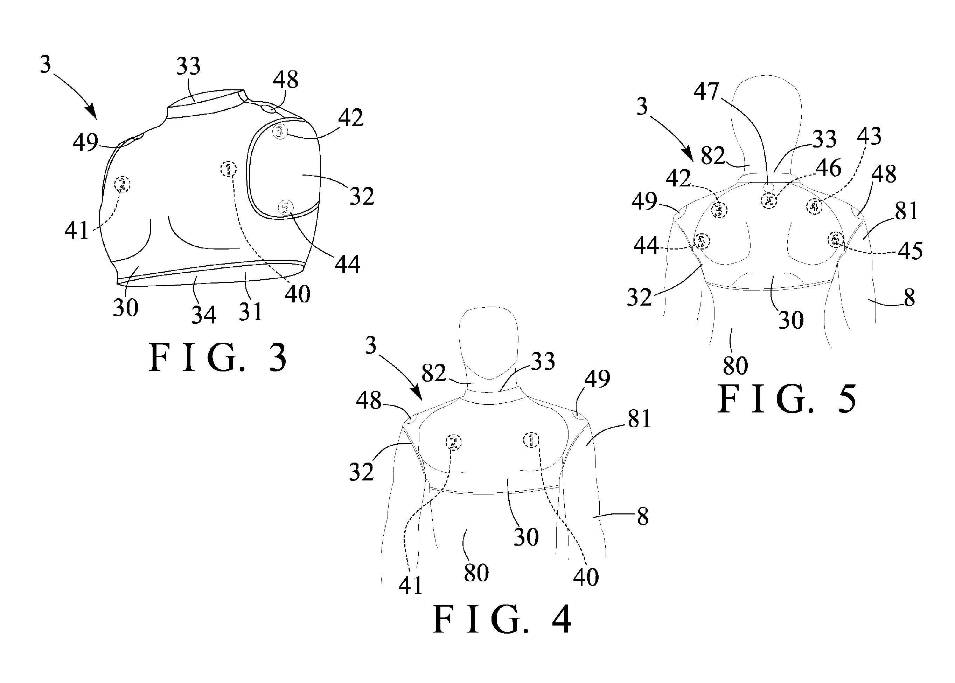

Referring to Fig. 1, a muscle activity training facility comprises a computer 10 including a processor 11, and a monitor 12 coupled to the processor 11 and having a screen 15 for showing data or information, such as the front and the rear view of the user 8 (Figs. 4-5), or the operations or exercises of the user 8 (Figs. 2, 6-9). An adaptor 3 includes a carrier 30 firmly attached to the upper body 80 of the user 8 (Figs. 4-5) even when the upper body 80 of the user 8 is exercising. The carrier 30 includes a chamber 31 (Fig. 3) for receiving the upper body 80 of the user 8, two side openings 32 for engaging with the arms 81, and an upper opening 33 for engaging with the neck 82.

A number of electrodes 40-45 (Figs. 1, 3-5) are attached to an inner portion 34 of the carrier 30 atselected locations, for example, the electrodes 40, 41 are disposed at the front portion of the carrier 30 for engaging with the chest of the upper body 80 of the user 8, and the other electrodes 42-45 are located at the other positions, such as the upper or shoulder portions of the upper body 80, or the side or the arm portion of the upper body 80. Another electrode 46, such as a G-sensor 46 (Figs. 1, 5) is attached to the inner portion 34 at the upper and back portion of the carrier 30, corresponding to the cervical vertebrae and the thoracic vertebrae of the user 8, such that the electrodes 40-46 may detect the muscle activities or the exercises of the muscles of the user 8, and are coupled to the processor 11. The adaptor 3 includes one or more (such as two) pointers 47-49 for aligning with selected portions of the user 8, such as the cervical vertebrae and the thoracic vertebrae, or the acromion processes of the user 8, etc. for allowing the electrodes 40-46 to be aligned with the selected muscles of the user 8.

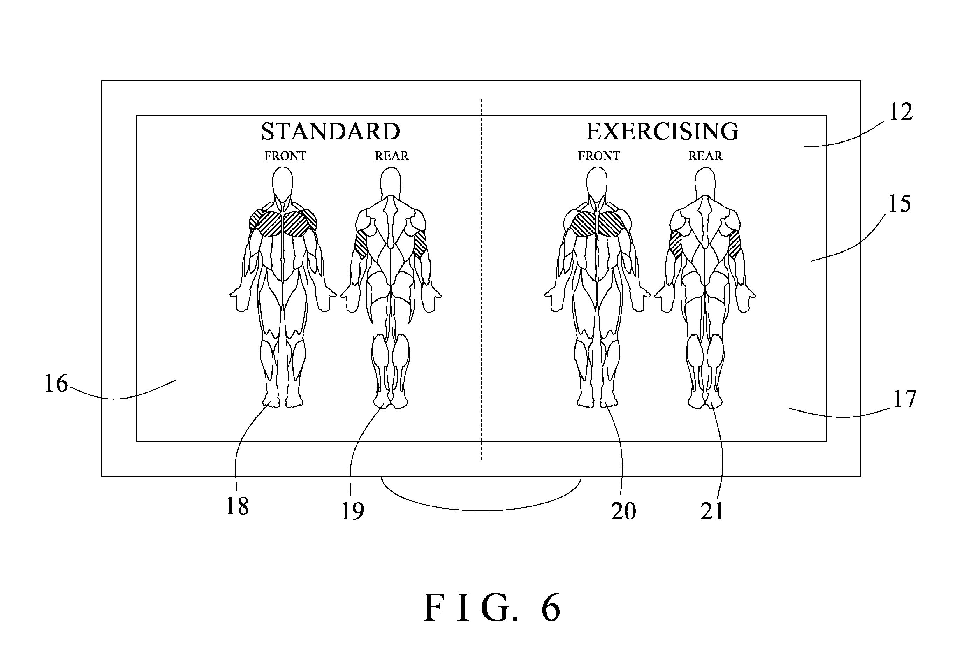

The screen 15 includes a left or first area 16 having two figures 18, 19 for showing the standard or correct operations of the muscles in the front and the rear portions of the user 8 that have been recorded or stored in the computer 10 and indicated or shown by shaded lines, different colors, the darkness, the brightness or the like. The right or second area 17 of the screen 15 includes two figures 20, 21 (Figs. 2, 6) for showing the exercising of the upper body 80 of the user 8 and the signals from the electrodes 40-46 and/or processed by the processor 11, the figures 20, 21 may be indicated or shown by shaded lines, different colors, the darkness, the brightness or the like.

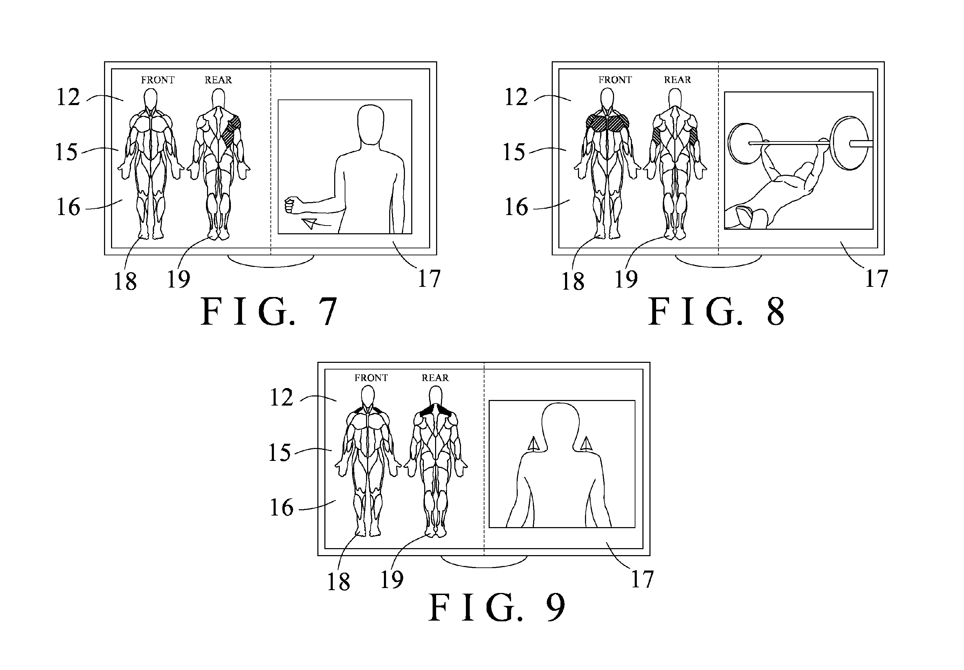

As shown in Figs. 7-9, the right or second area 17 of the screen 15 may also be used to show the actuating or exercising of the user 8, such as the shoulder or upper arm is outwardly rotated (Fig. 7), the pushing of the arms (Fig. 8), the elevating of the shoulder of the user 8 (Fig. 9). The muscles shown in the left area 16 of the screen 15 show that the muscles of the user 8 are properly or correctly used or operated, and recorded or stored in the computer 10 as the standard or correct information. The operations or activities of the other muscles of the user 8 that have not been shown in Figs. 7-9 may also be recorded or stored in the computer 10.

In operation, the carrier 30 may be attached to the upper body 80 of the user 8 (Figs. 4-5) by aligning the pointers 47-49 with the selected positions of the user 8, the user may then select the required activity (Fig. 2), such as the outwardly rotating of the shoulder or upper arm, which will be shown in the lower or selected area 22 of the screen 15, and/or shown in the right or second area 17 of the screen 15 (Figs. 7-9). When the user 8 is exercising, the figures 20, 21 may be shown on the right or second area 17 of the screen 15 (Figs. 2, 6) by shaded lines, different colors, the darkness, the brightness or the like for indicating the exercises of the muscles in the upper body 80 of the user 8. The user 8 may compare the figures 20, 21; 18, 19 to determine the difference between the muscles in order to correct the exercises of the user 8.

## Claims

1. A muscle activity training facility comprising a processor (11), a monitor (12) coupled to the processor (11) and having a screen (15), **characterized in that**:
the screen (15) includes a first area (16) and a second area (17), two first figures (18, 19) in the first area (16) for showing standard activities of muscles in front and rear portions of a user, two second figures (20, 21) in the second area (17) for showing operations of muscles in the front and the rear portions of the user, a carrier (30) attached to an upper body of the user, and a plurality of electrodes (40-45) attached to the carrier (30) and coupled to the processor (11).

2. A facility as claimed in claim 1, wherein the carrier (30) includes a chamber (31) for receiving the upper body of the user, two side openings (32) for engaging with upper arms (81), and an upper opening (33) for engaging with a neck (82) of the user.

3. A facility as claimed in claim 1 or 2, wherein the carrier (30) includes a G-sensor (46).

4. A facility as claimed in one of claims 1 to 3, wherein the monitor (12) includes a selected area (22) in the screen (15) to show the activities of the muscles of the user.

5. A facility as claimed in one of claims 1 to 4, wherein the carrier (30) includes at least one pointer (47-49).

6. A facility as claimed in one of claims 1 to5, wherein the carrier (30) is selected from a cloth.
